# FASCICULE DE BREVET EUROPEEN

(11) **EP 0 767 637 B1**
(45) Date de publication et mention de la délivrance du brevet: **25.11.1998**
(21) Numéro de dépôt: 95923403.0
(22) Date de dépôt: 16.06.1995
(51) Int. Cl.: A61F 2/44

(54) **PROTHESES DES FACETTES ARTICULAIRES VERTEBRALES**
WIRBELGELENKFORTSATZ-PROTHESE
VERTEBRAL ARTICULAR FACET PROSTHESES

(30) Priorité: 24.06.1994 FR 9407774
(43) Date de publication de la demande: 16.04.1997
(73) Titulaire: Fairant, Paulette, F-31170 Tournefeuille (FR); Martin, Jean-Raymond, F-31170 Tournefeuile (FR)
(72) Inventeur: MARTIN, Jean-Raymond, F-31170 Tournefeuille (FR)
(74) Mandataire: Cabinet BARRE LAFORGUE & associés
(86) Numéro de dépôt international: FR9500802
(87) Numéro de publication internationale: WO9600049

(56) Documents cités:
- EP-A- 0 408 489
- EP-A- 0 570 929
- DE-U- 9 304 368
- FR-A- 2 623 085
- US-A- 5 127 912

## Description

L'invention concerne des dispositifs de remplacement des surfaces articulaires des facettes vertébrales postérieures, atteintes par un processus de dégénerescence et/ou de destruction plus ou moins important, et des dispositifs d'ancrage de ces prothèses sur les vertèbres. Il s'agit d'éléments prothétiques formant un ensemble monobloc plaque de support-lame articulaire artificielle fixé sur la vertèbre correspondante par des moyens d'ancrage au niveau des pédicules et/ou des apophyses transverses et/ou de l'apophyse épineuse.

En rapport avec la dégénérescence des surfaces articulaires vertebrales, nous obtenons une arthrose des surfaces des facettes qui se manifeste par une réduction de l'épaisseur du cartilage pouvant aboutir à la disparition complète de ce dernier. De plus à la periphérie des surfaces articulaires érodées il existe une production ostéophytique susceptible d'engendrer des compressions neurologiques au niveau soit des trous de conjugaison soit du canal médullaire. Ces phénomènes sont responsables des lombalgies et des radiculalgies qui atteignent une partie importante de la population.

Le plus souvent nous sommes en face d'un processus dégénératif qui atteint essentiellement les surfaces articulaires mais nous pouvons avoir un processus plus invasif traumatique, infectieux, tumoral ou malformatif (spondylolisthésis, par exemple) entrainant la destruction de tout ou partie de l'apophyse articulaire.

Il existe donc une necessite de réduire et/ou de supprimer les frottements des surfaces articulaires degenérees: jusqu'à présent, ce but a été obtenu soit en supprimant tout mouvement des vertèbres impliquées par une arthrodèse ou fusion vertébrale plus ou moins associee à une ostéosynthèse (Harrington, Cotrel-Dubousset, TSRH, New Orleans, etc.) soit en réduisant ces mouvements par une ligamentoplastie intervertébrale (Graf. Sénégas Lemaire, etc.).

La suppression des mouvements intervertébraux, par arthrodèse ou par ligamentoplastie, conduit, outre à une rigidité vertébrale, à un report sur les facettes articulaires des étages vertébraux voisins sus- et sous-jacents des contraintes normalement absorbées par l'espace intervertébral considéré et donc à une accentuation de la dégénerescence de leurs facettes articulaires.

Quant aux protheses discales, elles réalisent un "espacement" de deux corps vertébraux tout en leur permettant de conserver ces mouvements. Elles apportent une solution au probleme de vieillissement du disque intervertébral mais elles n'ont aucun effet sur la réduction des efforts demandés aux facettes articulaires postérieures dont les mouvements et les frottements sont conservés.

L'invention vise donc à pallier les inconvénients de l'ensemble des dispositifs connus en proposant un nouveau dispositif qui réalise un remplacement des surfaces articulaires dégénérées par des prothèses articulaires. Ces prothèses vont conserver la mobilite inter-vertébrale tout en supprimant les frottements "osteo-cartilagineux" des surfaces usées et donc les douleurs et/ou la production d'ostéophytes susceptibles d'entrainer des compressions radiculaires dans les foramens.

L'invention concerne donc soit des éléments prothétiques de traitement de surface articulaire soit des éléments prothétiques plus importants réalisant un remplacement complet de toute l'apophyse osseuse et de sa surface articulaire. L'invention vise à conserver la stabilité de la colonne vertébrale par "l'accrochage" mécanique intervertébral que réalisent les éléments prothétiques des apophyses entre eux, comme le font les apophyses vertébrales naturelles, et à respecter le potentiel de mobilité fonctionnelle sans altérer l'appareil musculo-ligamentaire paravertébral.

Ces éléments prothétiques, réalisés en matériau biocompatible tel qu'un alliage metallique (acier inoxydable, titane, ou autre) et/ou un matériau composite, présentent une premiere face dite "articulaire" qui est en contact avec la surface similaire de la vertèbre adjacente afin de réaliser entre elles un glissement avec un minimum de frottement et une deuxieme face opposée dite "osseuse", poreuse, parsemee de picots, recouverte ou non d'hydroxyapatite, et destinée à venir en appui sur l'os restant de l'apophyse articulaire vertébrale. Ainsi selon l'invention ces éléments prothétiques intéressent au minimum les surfaces articulaires d'un espace intervertébral correspondant aux apophyses articulaires inférieures de la vertèbre sus-jacente et à celles supérieures de la vertèbre sous-jacente. Si necessaire les prothèses peuvent être unilatérales mais elles sont le plus souvent et de préférence bilatérales.

Si le capital osseux restant de l'apophyse articulaire est trop faible, la prothèse remplace l'entière apophyse et comporte donc toujours une surface dite "articulaire" de glissement mais la face opposée habituellement "osseuse" est remplacée par un épaississement de la prothèse, de manière à remplacer totalement l'apophyse articulaire naturelle dont elle reprend toutes les caractéristiques anatomiques quant à la forme, au volume et à l'orientatio, dont la surface est lisse et légèrement convexe pour éliminer tout risque pour les éléments médullaires ou neurologiques présents en dedans et en avant.

Etant donné d'une part la faible quantité d'os que représente l'arc postérieur de la vertèbre dont les apophyses articulaires font partie et d'autre part la qualité "spongieuse" c'est-à-dire fragile de cet os, se pose le problème majeur de l'ancrage de ces prothèses de facettes articulaires artificielles. L'ancrage doit en effet réaliser une stabilité parfaite de ces protheses articulaires qui seront soumises aux forces developpees lors des mouvements intervertébraux, puisqu'elles ont pour but de preserver ces mouvements intervertébraux.

L'invention vise donc à palier ces difficultes d'ancrage posées par le faible capital osseux que represente chaque facette articulaire en proposant un dispositif d'ancrage d'une prothèse de facette articulaire vertébrale, comprenant au moins un support portant des moyens de liaison de la prothèse à ce support et des moyens d'ancrage de ce support par rapport à la vertèbre, caractérisé en ce que ce support comporte une face convexe épousant et venant en appui au contact d'une portion au moins de la surface concave de l'arc postèrieur de la vertèbre d'un côté au moins de l'apophyse épineuse, et en ce que les moyens d'ancrage associent rigidement au moins une portion distincte du support à au moins une partie distincte correspondante de la vertèbre.

Ce dispositif comporte au moins un support dont la face convexe s'étend en regard ou de l'apopnyse epineuse et/ou une apophyse transverse et/ou un pédicule de la vertèbre, et des moyens d'ancrage de ce support respectivement ou sur l'apophyse épineuse et/ou une apophyse transverse et/ou un pédicule de la vertèbre. La vis intrapédiculaire lorsqu'elle n'est pas utilisée isolément n'est pas obligatoire et peut alors être éventuellement remplacée par une simple vis courte passant la corticale postérieure et s'arrêtant dans l'os spongieux en regard du pédicule: cela évite tout risque de lésion neurologique.

Chaque support est formé d'une plaque portant des moyens de couplage et recouvrant une portion de la surface concave de l'arc postérieur de la vertèbre d'un côté au moins de l'apophyse épineuse.

Un dispositif peut comporter deux supports, un de chaque côté de l'apophyse épineuse de la vertèbre, ou un seul support s'étendant d'un seul côte ou des deux côtés de l'apophyse épineuse. Avantageusement, les deux supports sont associés ensemble à la même apophyse epineuse par des moyens d'ancrage communs. L'apophyse epineuse, dont la corticale est entierement respectee, est ainsi étayee et realise donc l'appui et l'union des deux supports bilateraux. Les deux côtés de l'arc postérieur vertébral peuvent servir d'ancrage y compris pour une prothése unilatérale.

Les moyens d'ancrage de chaque support comportent en regard d'une apophyse osseuse transverse au moins un crochet articulé au support autour d'un axe, ce crochet portant une vis de serrage engagée dans un tarraudage du crochet, cette vis prenant appui directement ou indirectement sur le support ou sur un autre crochet pour faire pivoter le crochet autour de son axe dans le sens du serrage de l'extremite libre du crochet sur la partie correspondante de la vertèbre. Avantageusement, les moyens d'ancrage comportent au moins une pince de serrage formée de deux crochets articulés au même axe ou à des axes parallèles et dont les extrémités libres en regard se serrent l'une vers l'autre.

Les moyens d'ancrage de chaque support comportent au moins une bride de serrage d'une extrémité du support sur une apophyse vertébrale, notamment sur une apophyse épineuse et/ou transverse.

Par ailleurs, la face convexe du support est formee d'un metal poreux et avantageusement recouverte d'une couche d'hydroxyapatite. Cette face osseuse est parsemee de picots, de contours circulaires ou polyedriques, de 1 à 2mm de hauteur, destinés à assurer une meilleure implantation et fixation du support sur la corticale de l'arc postérieur vertébral.

Le moyen de liaison de la prothèse à son support est formé par la base séssile de cette prothèse: c'est-à-dire que cette base s'élargit progressivement de la lame prothetique vers le support de plaque tout en étant plus large et épaisse que cette lame prothetique qu'elle soutient. Cette base est en position supérieure ou inferieure et établit ainsi la jonction avec les moyens d'ancrage sur la vertèbre.

Selon l'invention, l'élément prothétique de l'apophyse articulaire vertébrale est caractérisé :
. en ce qu'il comporte :
   - au moins une plaque de support (7) postérieure adaptée pour être ancrée rigidement à une vertèbre en s'étendant contre une portion au moins d'un arc postérieur de la vertèbre,
   - au moins une lame prothètique (1, 11, 5) d'une facette articulaire, incluant ou non le remplacement total de l'apophyse articulaire (3, 13) vertébrale postérieure sur laquelle elle est adaptée à s'appuyer,
. et en ce que chaque lame prothétique (1, 11, 5, 15) est portée par la plaque de support (7), s'étend à partir de la plaque support (7) jusqu'à la position de la facette articulaire naturelle qu'elle remplace, et présente une surface articulaire artificielle (2, 12) dont les formes, la position et l'orientation correspondent à celles de la facette articulaire naturelle (3, 13) qu'elle remplace.

Selon un mode de réalisation de l'invention l'element prothétique est caracterisé en ce qu'il comporte au moins une plaque de support (7) qui porte la lame prothétique supérieure (1) à surface articulaire (2) plane ou concave orientée vers l'arrière, adaptée pour le remplacement d'une facette articulaire supérieure, et/ou une lame prothetique inferieure (11) à surface articulaire (12) plane ou convexe orientée vers l'avant, adaptée pour le remplacement d'une surface articulaire inferieure.

Selon un mode de réalisation de l'invention, l'element prothétique est caractérisé en ce articulaire, cette lame prothétique présentant une face opposee a la surface articulaire (2, 12) s'étendant contre une portion residuelle de l'apophyse articulaire (3, 13) correspondante.

Selon un mode de réalisation de l'invention, l'element prothétique est caractérisé en ce qu'il comporte au moins une lame prothétique (5, 15) de remplacement total d'une apophyse articulaire (3, 13), cette lame prothétique (5, 15) ayant une forme de tubérositè presentant une surface articulaire artificielle (2, 12), une protubérance renflee pour former, comme l'apophyse articulaire naturelle qu'elle remplace, une limite postèro-externe au canal medullaire et une limite postérieure au foramen.

Selon un mode de réalisation de l'invention, l'élément prothétique est caractérisé en ce que chaque lame prothétique (1, 11, 5, 15) est reliée à une plaque de support (7) par une base séssile (6, 16), l'ensemble de la lame prothétique (1, 11, 5, 15), de la plaque de support (7) et de la base séssile (6, 16) étant formé d'une seule pièce rigide en matériau biocompatible.

Selon un mode de réalisation de l'invention, l'élément prothétique est caractérisé en ce que chaque plaque de support (7) comprend une face convexe qui est adaptée pour épouser et venir en appui et au contact de la surface concave de l'arc vertébral postérieur, et en ce que chaque plaque de support (7) est une plaque mince adaptée pour ne pas interférer avec les organes naturels adjacents, notamment avec les muscles et les ligaments adjacents.

Selon un mode de réalisation de l'invention, l'élément prothétique est caractérisé en ce que, de chaque côté de l'apophyse épineuse (9) où une plaque de support (7) est prévue, les moyens d'ancrage rigide de la plaque de support (7) sont adaptés pour ancrer cette plaque de support (7) contre l'arc postérieur de la vertèbre :
- soit par rapport au pédicule (10) de l'arc posterieur en regard,
- soit par rapport au pédicule (10) et sur une apophyse transverse (8) ou épineuse (9) de la vertèbre,
- soit sur une apophyse transverse (8) et sur l'apophyse epineuse (9),
- soit sur une apophyse transverse (8), sur l'apophyse epineuse (9), et par rapport au pédicule (10) de l'arc posterieur en regard.

Selon un mode de réalisation de l'invention, l'élément prothétique est caracterisé en ce qu'il comporte une plaque de support (7) unilatérale s'étendant d'un côte seulement de l'apophyse épineuse (9) de la vertebre.

Selon un mode de réalisation de l'invention, l'élément prothétique est caracterisé en ce qu'il comporte une plaque de support (7) bilatérale s'étendant des deux côtés de l'apophyse épineuse (9) de la vertèbre.

Selon un mode de réalisation de l'invention, l'élément prothétique est caractérise en ce qu'il comporte deux plaques de support (7), une de chaque côté de l'apophyse epineuse (9) de la vertèbre.

Selon un mode de réalisation de l'invention, l'élement prothétique est caractérisé en ce que les deux plaques de support (7) s'étendent en regard de l'apophyse épineuse (9) et y sont ancrées par des moyens d'ancrage communs (21, 22).

Selon un mode de réalisation de l'invention, l'élement prothétique est caractérisé en ce qu'il comporte des moyens d'ancrage rigide d'une plaque de support (7) sur une apophyse transverse (8), qui sont constitues d'au moins un crochet (28) et/ou d'au moins une pince (33) et/ou d'au moins un collier (39) de serrage de la plaque de support (7) qui s'étend en regard de cette apophyse transverse (8).

Selon un mode de réalisation de l'invention, l'élement prothétique est caractérisé en ce que les moyens d'ancrage de la plaque de support (7) sur l'apophyse transverse (8) comportent au moins un crochet (28, 34, 35) mobile articule a la plaque de support (7) autour d'un axe (30, 36, 37), ce crochet (28, 34, 35) mobile portant une vis de serrage (32, 42, 43), engagee dans un taraudage du crochet (28, 34, 35), cette vis étant adaptée pour prendre appui directement ou indirectement sur la plaque de support (7) ou sur un autre crochet, pour faire pivoter le crochet (28, 34, 35) mobile autour de son axe (30, 36, 37) dans le sens du serrage de son extremité libre (44, 45), sur la partie correspondante de la vertébre.

Selon un mode de réalisation de l'invention, l'élement prothétique est caractérisé en ce que les moyens d'ancrage comportent au moins une pince (33) de serrage formée de deux crochets (34, 35) et dont les extrémités libres (44, 45) en regard se serrent l'une vers l'autre.

Selon un mode de réalisation de l'invention, l'élement prothétique est caractérisé en ce qu'il comporte des moyens d'ancrage rigide d'une plaque de support (7) sur une apophyse épineuse (9), qui sont constitués d'au moins un collier (21) et/ou d'au moins une bride (22) de serrage de la plaque de support (7) qui s'etend en regard de cette apophyse epineuse (9).

Selon un mode de réalisation de l'invention, l'élement prothétique est caractérisé en ce que chaque plaque de support (7) porte en outre des moyens de couplage (49) d'une instrumentation rachidienne.

Selon un mode de réalisation de l'invention, l'élement prothétique est caractérisé en ce que la face d'appui osseuse de chaque lame prothétique (1, 11, 5, 15) et/ou de chaque plaque de support (7) est formée de métal poreux parsemé de picots.

Selon un mode de réalisation de l'invention, l'élement prothétique est caractérisé en ce que la face d'appui osseuse de chaque lame prothétique (1, 11, 5, 15) et/ ou de chaque plaque de support (7) est recouverte d'une couche d'hydroxyapatite.

Selon un mode de réalisation de l'invention, la prothese articulaire vertébrale est caractérisée en ce qu'elle comporte un premier élément prothétique selon l'invention adapte pour être ancré sur la vertèbre superieure de l'articulation à traiter, cet élement prothétique supérieur comprenant une lame prothétique inférieure (11) présentant une surface articulaire artificielle inférieure (12), et un deuxième élément prothétique selon l'invention adapté pour être ancré sur la vertèbre inférieure de l'articulation à traiter, cet élément prothétique comprenant une lame prothétique supérieure (1) présentant une surface articulaire artificielle (2), lesdites surfaces articulaires artificielles inferieure et superieure ayant des formes conjuguées adaptées pour réaliser l'articulation des deux vertèbres.

D'autres caracteristiques, buts et avantages de l'invention ressortiront de la description détaillee qui suit, en référence aux dessins annexés dans lesquels:

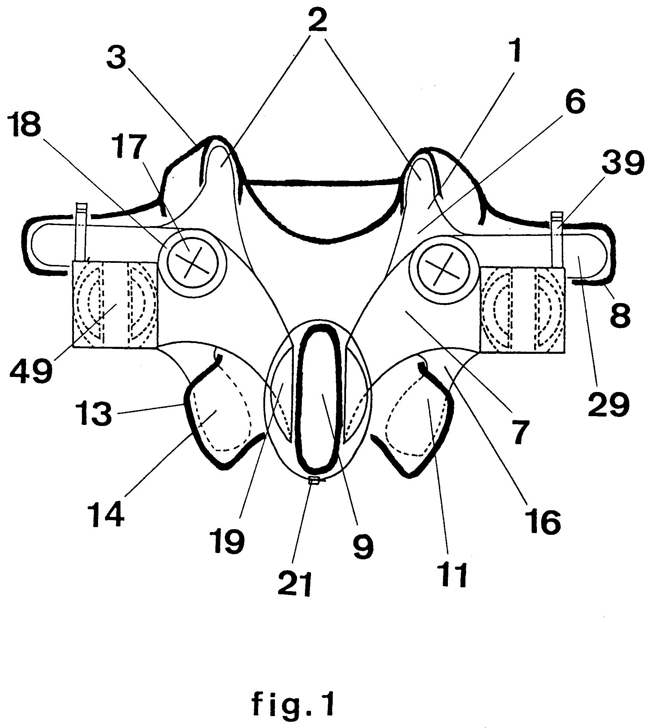

- la figure 1 est une vue schematique posterieure d'une prothése vertébrale implantee et réalisant le remplacement des surfaces articulaires des apophyses vertébrales supérieures et inférieures bilatéralement, et montrant l'association possible avec l'insertion d'une orthèse vertébrale implantée dynamique équipée de dispositifs d'ancrage selon l'invention.

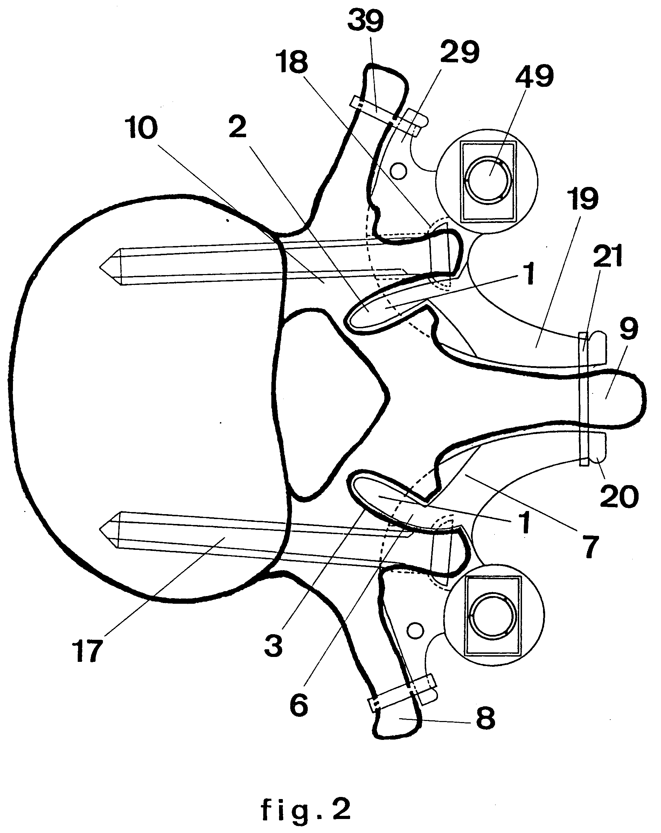

- la figure 2 est une vue schématique en section par un plan horizontal de la figure 1, montrant les prothèses supérieures et leurs moyens d'ancrage sur trois points que sont les pédicules, les apophyses transverses et les apophyses épineuses, dans une réalisation en association avec une orthèse vertebrale implantee dynamique.

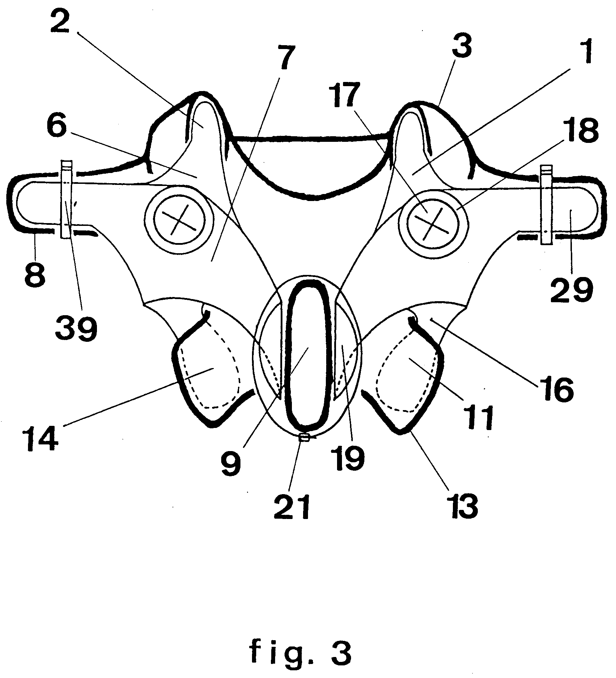

- la figure 3 est une vue schematique postérieure d'une prothese vertébrale implantée de maniere isolée et fixée sur les trois points d'ancrage habituels.
- la figure 4 est une vue schematique postérieure d'une prothese vertébrale implantée et fixée sur la vertèbre par deux points dans les pédicules et sur les apophyses épineuses.
- la figure 5 est une vue schématique postérieure d'une prothése vertébrale implantée et fixée sur la vertèbre par seulement une vis intrapédiculaire de chaque côté de l'apophyse épineuse.
- la figure 6 est une vue schematique posterieure d'une variante de réalisation dans laquelle la vertèbre ne comprend pas d'apophyse epineuse: la plaque est de type monobloc et passe en pont entre les deux fosses paravertébrales en recouvrant postérieurement le canal médullaire qu'elle protege.
- la figure 7 est une vue schematique supérieure d'une prothèse vertébrale implantée au niveau de la 12e vertébre dorsale qui ne presente pas d'apophyse transverse: une vis oblique en direction du pedicule stabilise la prothése.
- la figure 8 est une vue schématique postérieure de la prothése implantée au niveau de la lère vertèbre sacrée avec une plaque monobloc ancrée au niveau des pédicules et des lers trous de conjugaison.

Selon l'invention, la lame 1 de la prothése de la facette supérieure a la même orientation que l'articulation qu'elle remplace. Au niveau lombaire elle se situe dans un plan vertical et oblique par rapport au plan frontal et/ou sagittal. Sa surface 2 regarde en arriere et en dedans. L'angle voisin de 45° au niveau lombaire est variable avec le niveau vertébral considéré. Au niveau dorsal et cervical elle a une orientation plus frontale et oblique par rapport au plan horizontal. Sa surface 2 regarde alors en arrière et en haut.

La forme de la lame 1 de la prothèse est ovalaire à grand axe vertical. Sa surface 2 est généralement plane mais peut-être légèrement concave ( en concordance avec la surface 12 légèrement convexe de la lame 11 de la prothèse de l'apophyse articulaire inférieure 13 ) pour permettre certains degrés de mouvements de rotation selon le niveau vertébral considéré.

La lame 1 de la prothese remplace le cartilage dégéneré et va donc s'appuyer sur l'os restant de l'apophyse articulaire 3. Son épaisseur variable, de 2 à 3mm habituellement, peut être plus importante si la force demandée le justifie, et sa face non articulaire poreuse est plus ou moins recouverte d'hydroxy-apatite pour une meilleure fixation et integration à l'os restant.

Dans le cas où la fragilitè de l'os restant ou son absence justifie le remplacement total de l'apophyse 3, la lame de la prothése est épaissie pour supporter à elle seule tous les efforts demandes et reprend la forme anatomique de la totalité de l'apophyse articulaire 13 considérée: sa surface articulaire 2 reste identique à celle utilisée lors d'un remplacement partiel alors que la face 5 opposée est formée de matériau plein réalisant une face 5 convexe lisse qui sert de limite interne et posterieure au canal médullaire et de limite posterieure au foramen.

La surface 2 de glissement de cette lame 1 de la prothese est recouverte d'un materiau biocompatible à fort taux de glissement (ou a faible taux de frottement) tel que, et de manière non limitative, acier inoxydable, titane, ceramique, polyéthylène de haut poids moléculaire, ou matériau composite.
Elle va s'articuler avec la surface 12 ( de matériau identique ou différent ) de la lame 11 de la prothèse articulaire inférieure de la vertèbre supérieure. Ses dimensions respectent celles de la surface articulaire qu'elle remplace et peuvent même être réduites de 1 à 2mm en avant pour élargir le diamètre antéro-postérieur du foramen et donc éviter toute compression radiculaire.

La liaison de la lame 1 de la prothèse avec ses moyens d'ancrage se fait par l'élargissement progressif de sa base 6 qui vient former un monobloc avec son support 7. Cette base 6 sessile est convexe et poreuse sur sa face posterieure pour s'appliquer et se solidariser à l'os de l'arc vertébral sur lequel elle s'appuie. Le support 7 est appliqué contre la face postérieure de l'arc vertébral posterieur, en epousant ses contours : ce support ou plaque 7 est fixé à l'os par au moins l'un de ses moyens d'ancrage sur les apophyses transverses 8, épineuses 9 et/ou les pédicules 10.

Si la lame 1 de cette prothèse supérieure est placee sur la vertèbre inférieure du montage prévu, elle reste isolée sur cette vertèbre tout en utilisant selon les besoins un ou plusieurs moyens d'ancrage prévus. Dans le cas où la lame 1 de cette prothèse supérieure est posée sur une vertèbre "intermédiaire" du montage, elle s'associe alors à la lame 11 de la prothèse inférieure avec laquelle elle forme un monobloc par l'intermédiaire du support 7 qui réalise ainsi la même fixation vertébrale pour les deux lames prothétiques 1 et 11.

La lame 11 de la prothèse de l'apophyse articulaire inférieure 13 a la même orientation que la surface articulaire qu'elle remplace, c'est-a-dire qu'elle se situe dans un plan vertical et oblique par rapport au plan frontal et/ou sagittal. L'angle, voisin de 45° au niveau lombaire, est variable selon le niveau vertébral considéré et reste strictement parallèle à celui de la lame 1 de la prothèse de l'apophyse articulaire supérieure 3 de la vertèbre inférieure sur laquelle elle va s'appuyer afin de permettre des glissements avec un minimum de frottements.

La forme de la lame 11 de cette prothèse est ovalaire à grand axe vertical. Sa surface 12 est généralement plane mais peut-être légèrement convexe pour épouser la concavite de la lame 1 de l'apophyse articulaire supérieure 3 correspondante. Elle regarde en avant et en dehors pour s'appuyer sur la surface 2 de la lame 1 de la prothése de l'apophyse articulaire supérieure 3 de la vertèbre inférieure. Ses dimensions respectent celles de la surface articulaire qu'elle remplace et peuvent même être réduites de 1 à 2mm en avant pour élargir le diamètre antéro-postérieur du foramen et donc éviter toute compression radiculaire.

De manière similaire à la lame 1 de la prothèse de l'apophyse articulaire supérieure 3, cette lame 11 prothétique de l'apophyse articulaire inférieure 13 s'appuie sur l'os restant de l'apophyse articulaire 13 ou bien elle remplace complètement cette apophyse articulaire 13. Sa face celles de la lame 1 de la prothèse de l'apophyse articulaire supérieure 3, adaptees à l'anatomie de la vertèbre considérée.

Dans le cas ou la fragilite de l'os restant ou son absence justifie le remplacement total de l'apophyse articulaire inferieure 13, la lame 11 de la prothèse reprend la forme anatomique de cette apophyse articulaire inférieure 13: Sa surface articulaire 12 reste identique a celle utilisée lors d'un remplacement partiel alors que la face opposee 15 est formee de matériau plein réalisant une face convexe lisse qui sert de limite externe et postérieure au canal médullaire et de limite postérieure au foramen.

La lame 11 de la prothèse de l'apophyse articulaire inférieure se solidarise avec ses moyens d'ancrage en s'élargissant en une base 16 séssile qui est supérieure et qui effectue un mouvement d'enroulement externe autour de la base osseuse de l'apophyse articulaire inférieure 13 qui occupe une position postérieure et interne par rapport à la lame 11 de la prothèse de l'apophyse articulaire inférieure 13.

Avantageusement, dans le cas où sur une même vertèbre deux lames prothétiques articulaires supérieure 1 et inferieure 11 sont souhaitées, elles forment un "monobloc" avec le support 7 d'ancrage vertébral qui est commun: ceci a pour effet de renforcer leur stabilité puisqu'elles utilisent les mêmes moyens de fixation.

Quand la lame 11 prothétique de l'apophyse articulaire inférieure 13 est implantée sur la vertèbre la plus supérieure du montage choisi, elle est montée de manière isolée sur cette vertèbre et s'appuie donc par sa base 16 séssile supérieure sur son support 7 qui lui permet un ancrage vertébral sur au moins un des trois points que sont les apophyses transverses 8, epineuses 9 et les pédicules 10.

Avantageusement, les lames prothétiques 1 et 11 des articulations vertébrales postérieures peuvent être implantées isolément ou associées à une orthèse vertébrale interne dynamique et/ou à une ou plusieurs prothèses de disques intervertébraux qui vont réduire les forces appliquees à ces protheses articulaires.

Les dispositifs d'ancrage des lames prothétiques 1 et 11 comportent des supports 7 et des moyens d'ancrage sur au moins une portion distincte de chaque vertèbre. Ainsi, chaque support ou plaque 7 des dispositifs d'ancrage s'étend en regard du pédicule 10 de la vertèbre, et peut être fixé à la vertèbre sur au moins un pédicule 10 par une vis intra-pédiculaire 17 à tête fraisée engagée dans un perçage 18 correspondant de la plaque 7. Selon l'évaluation des risques neurologiques, la vis intrapédiculaire 17 peut être très courte, prenant toujours la direction du pédicule 10 , mais se limitant en profondeur à la corticale de l'arc postérieur et à l'os spongieux sous-jacent en regard du pédicule 10 mais sans pénétrer dans ce dernier afin d'éviter toute protrusion dans le canal médullaire ou le foramen.

Chaque plaque 7 des dispositifs d'ancrage s'étend aussi avantageusement en regard des faces latérales de l'apophyse épineuse 9, de chaque côté, et comporte également des moyens d'ancrage sur cette apophyse épineuse 9. Pour ce faire, l'extrémité 19 de la plaque venant en regard de l'apophyse epineuse 9 comporte un renflement 20 en saillie dans le plan frontal et dans la direction horizontale de manière à former un épaulement de retenue d'un cerclage qui peut être constitué par un collier 21 autobloquant, métallique ou en matériau synthétique, ou d'une bride 22 entourant l'apophyse épineuse 9 et serrant l'extrémité de la plaque 19. La bride 22 peut être constituée d'un cadre 23 entourant l'apophyse épineuse 9 et l'extremité de la plaque 19, d'une vis 24 à axe vertical engagee à travers un taraudage vertical du cadre, et dont l'extremité libre est munie d'un patin 25 prenant appui sur la face supérieure de l'apophyse épineuse 9. Ainsi le cadre 23 est bloque par rapport à l'apophyse épineuse 9. La bride 22 comporte également une vis 26 à axe horizontal frontal engagée dans un taraudage horizontal du cadre 23, et dont l'extrémité libre est munie d'un patin 27 qui prend appui sur l'extremité 19 de la plaque pour la serrer lateralement et horizontalement. contre l'apophyse épineuse 9. Après serrage de la vis 26, l'epaulement de retenue formé par le renflement bloque la bride 22 en translation horizontale par rapport à l'extrémité libre 19 de la plaque, le patin 27 venant en avant et contre ce renflement. La bride 22 associe ensemble les deux plaques 7 et constitue ainsi des moyens d'ancrage communs de ces deux plaques 7 à l'apophyse épineuse 9 de la vertèbre.

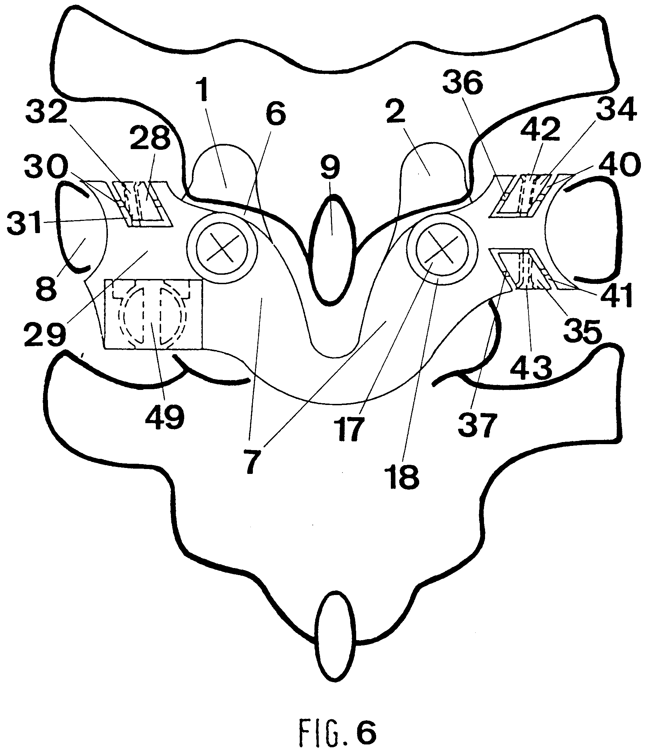

Egalement, chaque plaque 7 des dispositifs d'ancrage s'étend avantageusement en regard d'au moins une apophyse transverse 8 de la vertèbre, et comporte des moyens d'ancrage sur une apophyse transverse 8. Ces moyens d'ancrage peuvent être formés par au moins un crochet 28 ( plaque de gauche sur la figure 6) articulé à l'extremité transverse 29 de la plaque 7 autour d'un axe horizontal 30 et prenant appui sur la face supérieure et/ou sur la face inférieure de l'apophyse transverse 8. L'extrémité transverse 29 de la plaque 7 présente donc, pour chaque crochet 28, une chape 31 dans laquelle le crochet 28 est articulé autour de son axe 30. Chaque crochet 28 est muni, à son extrémite opposee à l'apophyse transverse 8, d'une vis verticale 32 engagée dans un taraudage du crochet 28 et prenant appui sur une surface horizontale du fond de la chape 31 de la plaque 7 pour serrer ce crochet 28 contre la surface correspondante de l'apophyse transverse 8.

Sur la figure 6, les moyens d ancrage de la plaque 7 de droite comportent une pince de serrage formee de deux crochets 34 et 35 articulés par rapport à la plaque 7 autour de deux axes 36 et 37 parrallèles horizontaux. En variante, l'ancrage sur l'apophyse transverse 8 peut être realise par un collier 39 autobloquant de serrage ( similaire a celui 21 utilisé sur l'apophyse épineuse 9) métallique ou de materiau synthétique. Chaque crochet 34 et 35 est similaire au crochet 28 sus-décrit et est monté dans une chape 40 et 41 de la plaque 7 avec chacun une vis verticale 42 et 43 de serrage prenant appui sur une surface horizontale du fond de la chape 40 et 41 pour serrer ce crochet 34 et 35 contre la surface correspondante respectivement supérieure et inférieure de l'apophyse transverse 8. Les extrémites libres respectives des deux crochets s'étendent en regard l'une de l'autre en opposition et se serrent l'une vers l'autre sous l'effet des vis 42 et 43 pour emprisonner l'apophyse transverse 8. A la place des deux vis 42 et 43 on peut prévoir un tendeur à deux pas de vis inversés reliant les deux crochets 34 et 35 l'un à l'autre et qui est accessible par le haut ou par le bas pour serrer ou desserrer les deux crochets 34 et 35. Une telle pince peut être prevue aussi pour la plaque 7 de gauche.

La figure 6 represente une variante de réalisation dans laquelle la plaque 7 s'étend dans les deux gouttières paravertébrales. la vertébre ne comportant pas d'apophyse épineuse 9. La plaque 7 est ancrée sur les deux apophyses transverses 8 et sur les deux pédicules 10. La plaque forme un pont recouvrant l'arc vertébral postérieur restant et protégeant le canal médullaire.

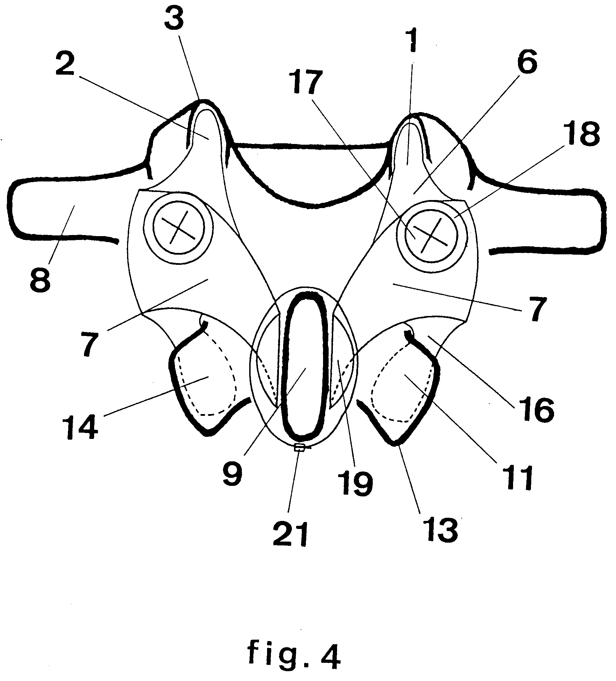

La figure 4 représente une autre variante dans laquelle les plaques 7 sont ancrées uniquement par les vis intrapédiculaires 17 et par la bride 22 sur l'apophyse épineuse 9.

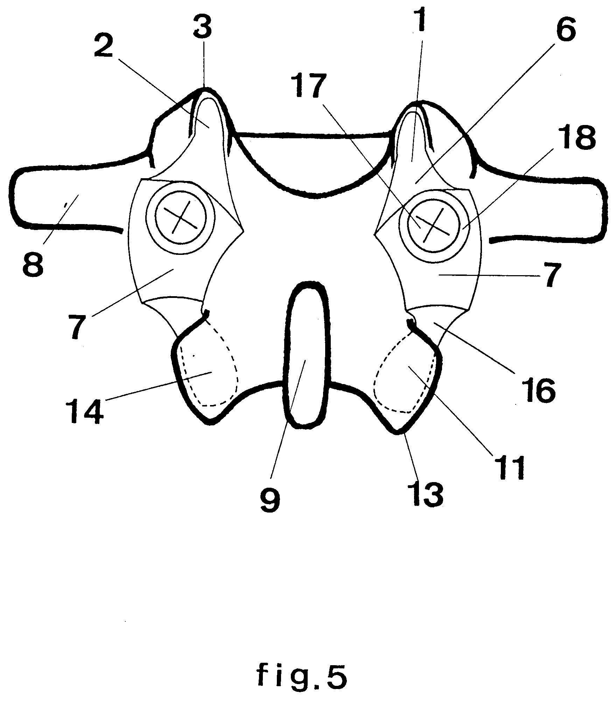

La figure 5 represente une autre variante dans laquelle les plaques 7 sont ancrées uniquement par les vis intrapédiculaires.

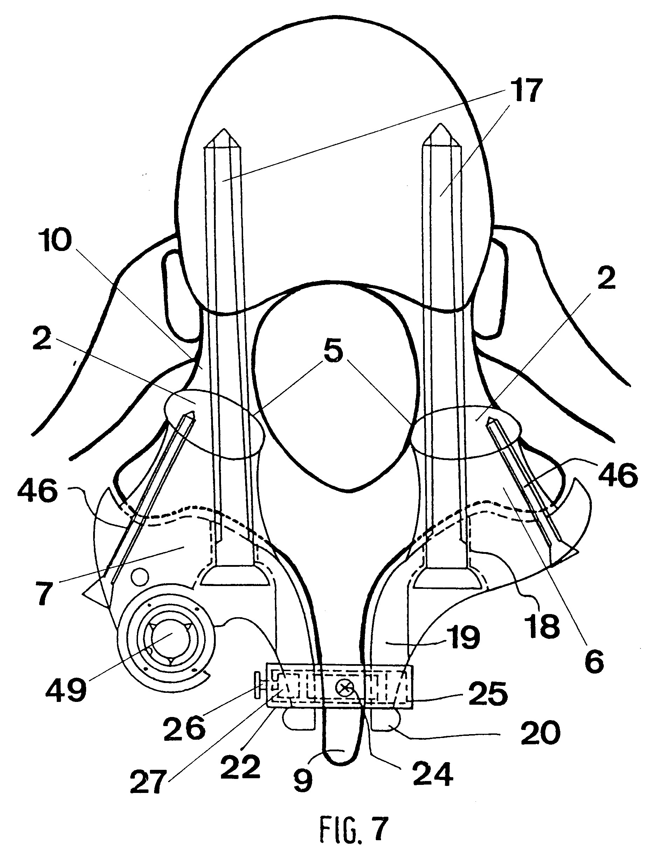

La figure 7 represente une autre forme de réalisation plus spécifiquement destinée à la vertebre dorsale D12 qui en général ne comporte pas d'apophyse transverse. Chaque plaque 7 epouse néammoins la forme du moignon de l'apophyse transverse 8 sur lequel elle est fixée par une vis oblique 46 orientée en direction du pédicule 10. Les plaques 7 sont aussi fixées par des vis intrapédiculaires 17 et une bride 22 de serrage sur l'apophyse épineuse 9.

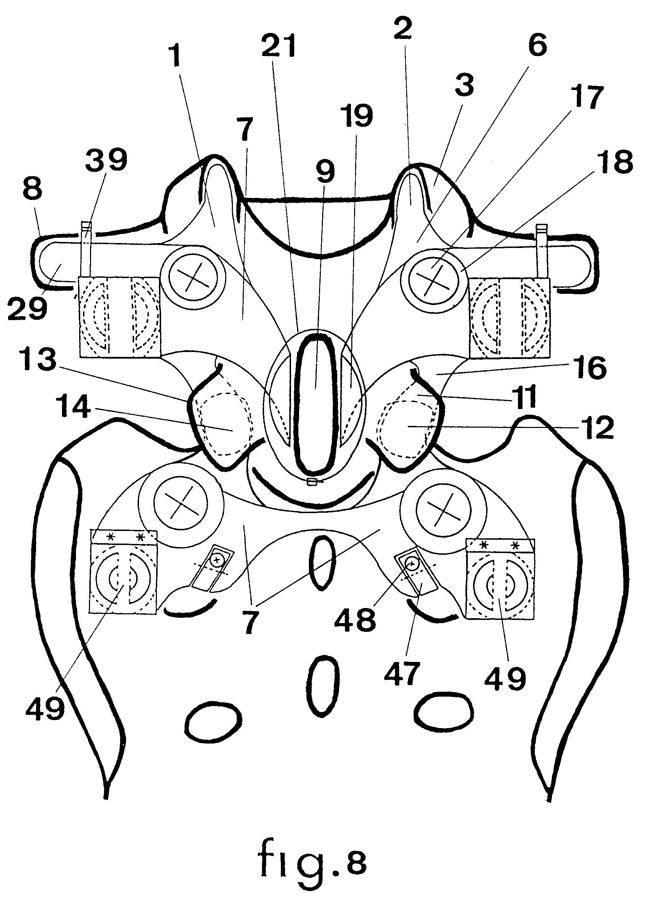

La figure 8 représente le cas de la vertébre sacrée S1 qui ne presente pas d'apophyse transverse mais un aileron sacré, on utilise une plaque 7 d'un seul tenant s'étendant des deux côtes de l'apophyse épineuse 9 hypotrophique en formant un pont au-dessus de cette apophyse epineuse 9. La plaque 7 peut être ancree par deux crochets inferieurs 47 similaires aux crochets des apophyses transverses 28 prealablement decrits, et qui s'appuient sur les bords supérieurs des deux premiers trous sacres. Chaque crochet 47 est serre par une vis de serrage 48 engagee dans un taraudage du crochet 47 et prenant appui sur une face verticale de la plaque 7. La plaque 7 est fixée sur la vertébre S1 par deux vis intrapédiculaires 17 passant par l'orifice 18 prévu dans la plaque et dirigées dans le corps vertébral vers le plateau superieur de S1. Deux autres vis (non représentees) peuvent stabiliser la plaque en la fixant lateralement à l'aileron sacré.

La technique chirurgicale de mise en place de ces prothèses est celle d'un abord classique postérieur de la colonne vertébrale avec ouverture des 2 fosses musculaires paravertébrales tout en conservant l'appareil ligamentaire intervertébral.

Les articulations sont ouvertes avec ablation des capsules articulaires puis des surfaces ostéocartilagineuses restantes des facettes vertebrales. Les ostéophytes marginaux sont reseques surtout afin de recalibrer en avant le foramen et en dedans le canal médullaire et donc éliminer toute compression neurologique comme dans le cas d'un canal lombaire étroit. Si la résection de l'os necessaire à l'insertion de la prothèse laisse un moignon apophysaire trop fragile, il est préférable d'en réaliser l'ablation et de procéder alors à l'insertion d'une prothèse totale de l'apophyse articulaire.

La prothese totale ou seulement articulaire de la facette vertébrale est alors choisie, en fonction du niveau instrumente, et positionnée; sa taille et son orientation sont vérifiées afin de bien correspondre à celle qui va lui être opposée.

La fixation est effectuée, en fonction des conditions anatomiques rencontrées, selon differentes associations sur les trois points d'ancrage que sont les apophyses épineuses, les apophyses transverses et les pédicules. Selon l'évaluation des risques neurologiques, la vis dite "intrapédiculaire" peut être tres courte, se limitant à la corticale posterieure et à l'os spongieux sous-jacent, en regard du pédicule et sans y pénétrer, afin d'éviter toute protrusion dans le canal medullaire ou le foramen: elle s'associe alors le plus souvent a un ancrage sur l'apophyse transverse et/ou épineuse afin d'assurer la stabilite du montage.

L'intervention se termine par la fermeture du site opératoire ou peut se poursuivre par la mise en place d'une instrumentation rachidienne dynamique afin de réduire les efforts demandes à ces protheses de facettes articulaires et aux autres structures paravertebrales. Eventuellement selon les cas, les disques intervertébraux peuvent bénéficier de la mise en place de prothèses discales permettant d'amortir les efforts demandes antérieurement à la colonne vertebrale (et donc en partie aux facettes articulaires postérieures) tout en lui conservant la plus grande mobilité possible.

## Revendications

1. Element prothétique d'apophyse articulaire vertébrale caractérise :
. en ce qu'il comporte :
- au moins une plaque de support (7) postérieure adaptee pour être ancrée rigidement à une vertèbre en s'étendant contre une portion au moins d'un arc postérieur de la vertébre,
- au moins une lame prothetique (1, 11, 5) d'une facette articulaire, incluant ou non le remplacement total de l'apophyse articulaire (3, 13) vertébrale postérieure sur laquelle elle est adaptée à s'appuyer.
. et en ce que chaque lame prothétique (1, 11) est portée par le plaque de support (7), s'étend à partir de la plaque de support (7) jusqu'à la position de la facette articulaire naturelle qu'elle remplace, et présente une surface articulaire artificielle (2, 12) dont les formes, la position et l'orientation correspondent à celles de la facette articulaire naturelle (3, 13) qu'elle remplace.

2. Elément selon la revendication 1, caractérisé en ce qu'il comporte au moins une plaque de support (7) qui porte la lame prothétique supérieure (1) à surface articulaire (2) plane ou concave orientée vers l'arrière, adaptée pour le remplacement d'une facette articulaire supérieure, et/ou une lame prothétique inférieure (11) à surface articulaire (12) plane ou convexe orientée vers l'avant, adaptée pour le remplacement d'une surface articulaire inférieure

3. Elément selon l'une des revendications 1 et 2, caractérise en ce qu'il comporte au moins une lame prothétique (1, 11) adaptée pour venir en appui contre une portion résiduelle d'apophyse articulaire, cette lame prothétique présentant une face opposée à la surface articulaire (2, 12) s'étendant contre une portion résiduelle de l'apophyse articulaire (3, 13) correspondante.

4. Element selon l'une des revendications 1 à 3, caractérisé en ce qu'il comporte au moins une lame prothétique (5, 15) de remplacement total d'une apophyse articulaire (3, 13), cette lame prothétique (5, 15) ayant une forme de tubérosité presentant une surface articulaire artificielle (2, 12), une protubérance renflée pour former, comme l'apophyse articulaire (3, 13) naturelle qu'elle remplace, une limite postéro-externe au canal médullaire et une limite postérieure au foramen.

5. Element selon l'une des revendications 1 à 4, caractérisé en ce que chaque lame prothétique (1, 11, 5, 15) est reliée à une plaque de support (7) par une base sessile (6, 16), l'ensemble de la lame prothétique (1, 11, 5, 15), de la plaque de support (7) et de la base séssile (6, 16) etant formé d'une seule piece rigide en matériau biocompatible.

6. Elément selon l'une des revendications 1 à 5 caractérisé en ce que chaque plaque de support (7) comprend une face convexe qui est adaptée pour épouser et venir en appui et au contact de la surface concave de l'arc vertébral postérieur, et en ce que chaque plaque de support (7) est une plaque mince adaptée pour ne pas interférer avec les organes naturels adjacents, notamment avec les muscles et les ligaments adjacents.

7. Element selon l'une des revendications 1 à 6, caractérisé en ce que, de chaque côté de l'apophyse épineuse (9) où une plaque de support (7) est prévue, les moyens d'ancrage rigide de la plaque de support (7) sont adaptés pour ancrer cette plaque de support (7) contre l'arc posterieur de la vertèbre :
- soit par rapport au pedicule (10) de l'arc postérieur en regard,
- soit par rapport au pédicule (10) et sur une apophyse transverse (8) ou épineuse (9) de la vertèbre.
- soit sur une apophyse transverse (8) et sur l'apophyse épineuse (9),
- soit sur une apophyse transverse (8), sur l'apophyse épineuse (9), et par rapport au pédicule (10) de l'arc postérieur en regard.

8. Element selon l'une des revendications 1 à 7, caractérisé en ce qu'il comporte une plaque de support (7) unilatérale s'étendant d'un côté seulement de l'apophyse épineuse (9) de la vertèbre.

9. Element selon l'une des revendications 1 à 7, caractérisé en ce qu'il comporte une plaque de support (7) bilatérale s'étendant des deux côtés de l'apophyse épineuse (9) de la vertèbre.

10. Elément selon l'une des revendications 1 à 8, caractérisé en ce qu'il comporte deux plaques de support (7), une de chaque côté de l'apophyse épineuse (9) de la vertèbre.

11. Elément selon la revendication 10, caractérisé en ce que les deux plaques de support (7) s'étendent en regard de l'apophyse épineuse (9) et y sont ancrées par des moyens d'ancrage communs (21, 22).

12. Elément selon l'une des revendications 1 à 11, caracterisé en ce qu'il comporte des moyens d'ancrage rigide d'une plaque de support (7) sur une apophyse transverse (8), qui sont constitués d'au moins un crochet (28) et/ou d'au moins une pince (33) et/ou d'au moins un collier (39) de serrage de la plaque de support (7) qui s'étend en regard de cette apophyse transverse (8).

13. Elément selon la revendication 12, caractérisé en ce que les moyens d'ancrage de la plaque de support (7) sur l'apophyse transverse (8) comportent au moins un crochet (28, 34, 35) mobile articulé à la plaque de support (7) autour d'un axe (30, 36, 37), ce crochet (28, 34, 35) mobile portant une vis de serrage (32, 42, 43), engagée dans un taraudage du crochet (28, 34, 35), cette vis étant adaptee pour prendre appui directement ou indirectement sur la plaque de support (7) ou sur un autre crochet, pour faire pivoter le crochet (28, 34, 35) mobile autour 0e son axe (30, 36, 37) dans le sens du serrage de son extremite libre, sur la partie correspondante de la vertèbre.

14. Element selon la revendication 13, caractérisé en ce que les moyens d'ancrage comportent au moins une pince (33) de serrage formée de deux crochets (34, 35) et dont les extrémités libres (44, 45) en regard se serrent l'une vers l'autre

15. Elément selon l'une des revendications 1 à 11, caractérisé en ce qu'il comporte des moyens d'ancrage rigide d'une plaque de support (7) sur une apophyse épineuse (9), qui sont constitues d'au moins un collier (21) et/ou d'au moins une bride (22) de serrage de la plaque de support (7) qui s'étend en regard de cette apophyse épineuse (9).

16. Elément selon l'une des revendications 1 à 15, caracterise en ce que chaque plaque de support (7) porte en outre des moyens de couplage (49) d'une instrumentation rachidienne.

17. Elément selon l'une des revendications 1 à 16, caractérisé en ce que la face d'appui osseuse de chaque lame prothétique (1, 11, 5, 15) et/ou de chaque plaque de support (7) est formée de métal poreux parsemé de picots.

18. Elément selon l'une des revendications 1 à 17, caractérisé en ce que la face d'appui osseuse de chaque lame prothétique (1, 11, 5, 15) et/ ou de chaque plaque de support (7) est recouverte d'une couche d'hydroxyapatite.

19. Prothése articulaire vertébrale caractérisée en ce qu'elle comporte un premier élément prothétique selon l'une des revendications 1 à 17, adapte pour être ancre sur la vertèbre superieure de l'articulation à traiter, cet élement prothetique supérieur comprenant une lame prothétique inférieure (11) présentant une surface articulaire artificielle inférieure (12), et un deuxième élément prothétique selon l'une des revendications 1 à 17, adapté pour être ancré sur la vertèbre inférieure de l'articulation à traiter, cet élément prothétique comprenant une lame prothétique supérieure (1) presentant une surface articulaire artificielle (2), lesdites surfaces articulaires artificielles inférieure et superieure ayant des formes conjuguées adaptées pour réaliser l'articulation des deux vertèbres.

## Claims

1. Prosthetic element for a vertebral articular apophysis, characterized in that it comprises:
- at least one posterior support plate (7) adapted to be rigidly anchored to a vertebra in a position in which it extends against a portion of at least one posterior arch of the vertebra,
- at least one prosthetic web (1, 11, 5) for an articular facet, either including or not including the whole replacement of the posterior vertebral articular apophyses (3, 13) on which it is adapted to be supported,
and in that each prosthetic web (1, 11) is carried by the support plate (7), extends from the support plate (7) to the position of the natural articular facet replaced by said prosthetic web, and has an artificial articular surface (2, 12) the shape, position and orientation of which correspond to those of the natural articular facet (3, 13) replaced by said prosthetic web.

2. Element according to claim 1, characterized in that it comprises at least one support plate (7) carrying the upper prosthetic web (1) having a flat or backwards directed concave articular surface (2), adapted for replacing an upper articular facet, and/or a lower prosthetic web (11) having a flat or forward directed convex articular surface (12) adapted for the replacement of a lower articular surface.

3. Element according to one of the claims 1 and 2, characterized in that it comprises at least one prosthetic web (1, 11) adapted to be supported against a residual portion of an articular apophysis, said prosthetic web having a face which is opposed to the articular surface (2, 12) extending against a residual portion of the corresponding articular apophysis (3, 13).

4. Element according to one of the claims 1 to 3, characterized in that it comprises at least one prosthetic web (5, 15) for replacing an articular apophysis (3, 13) totally, said prosthetic web (5, 15) having the form of a tuberosity with an artificial articular surface (2, 12), one swelling protuberance for forming, as the natural articular apophysis (3, 13) replaced by said prosthetic web, a posterior external limit for the medullary channel and a posterior limit for the foramen.

5. Element according to one of the claims 1 to 4, characterized in that each prosthetic web (1, 11, 5, 15) is linked to a support plate (7) by means of a sessile base (6, 16), wherein the assembly comprised of the prosthetic web (1, 11, 5, 15), the support plate (7) and the sessile base (6, 16) consists of one single rigid part made from a biocompatible material.

6. Element according to one of the claims 1 to 5, characterized in that each support plate (7) comprises a convex face which is adapted to accommodate and engage and be supported by the concave surface of the posterior vertebral arch, and in that each support plate (7) is a thin plate adapted so as not to interfere with the adjacent natural organs, particularly with the adjacent muscles and ligaments.

7. Element according to one of the claims 1 to 6, characterized in that on either side of the spinous apophysis (9) where a support plate (7) is provided, the means for rigidly anchoring the support plate (7) are adapted to anchor said support plate (7) against the posterior arch of the vertebra,
- either with respect to the pedicle (10) of the opposed posterior arch,
- either with respect to the pedicle (10) and on a transverse (8) or spinous (9) apophysis of the vertebra,
- either on a transverse apophysis (8) and on the spinous apophysis (9),
- or on a transverse apophysis (8), on the spinous apophysis (9), and with respect to the pedicle (10) of the opposed posterior arch.

8. Element according to one of the claims 1 to 7, characterized in that it comprises a one-sided support plate (7) extending only on one side of the spinous apophysis (9) of the vertebra.

9. Element according to one of the claims 1 to 7, characterized in that it comprises a two-sided support plate (7) extending on both sides of the spinous apophysis (9) of the vertebra.

10. Element according to one of the claims 1 to 8, characterized in that it comprises two support plates (7), namely one on each side of the spinous apophysis (9) of the vertebra.

11. Element according to claim 10, characterized in that both support plates (7), extend opposite the spinous apophysis (9) and are anchored therein by means of common anchoring means (21, 29).

12. Element according to one of the claims 1 to 11, characterized in that it comprises means for rigidly anchoring a support plate (7) on a transverse apophysis (8), wherein said means are comprised of at least one hook (28) and/or at least one clamping clip (33) and/or at least one collar (39) for clamping the support plate (7) extending opposite said transverse apophysis (8).

13. Element according to claim 12, characterized in that the means for anchoring the support plate (7) on the transverse apophysis (8) comprise at least one movable hook (28, 24, 35) hinged on the support plate (7) about an axis (30, 36, 37), wherein said movable hook (28, 34, 35) holds a clamping screw (32, 42, 43) which is engaged in a thread on the hook (28, 34, 35), said screw being adapted to be supported directly or indirectly on the support plate (7) or on another hook in order to pivot the movable hook (28, 24, 35) about the axis (30, 36, 37) thereof, in the direction of clamping of the free end thereof on the corresponding portion of the vertebra.

14. Element according to claim 13, characterized in that the anchoring means comprise at least one clamping clip (33) which is formed of two hooks (34, 35), and the opposed free ends (44, 45) thereof are clamped in direction of one another.

15. Element according to one of the claims 1 to 11, characterized in that it comprises means for rigidly anchoring a support plate (7) on a spinous apophysis (9), wherein said means are comprised of at least one collar (21) and/or at least one flange (22) for clamping the support plate (7) extending opposite said spinous apophysis (9).

16. Element according to one of the claims 1 to 15, characterized in that each support plate (7) holds means (49) for the coupling of a rachidian instrumentation.

17. Element according to one of the claims 1 to 16, characterized in that the bone support face of each prosthetic web (1, 11, 5, 15) and/or of each support plate (7) is formed of a porous metal provided with distributed trickles.

18. Element according to one of the claims 1 to 17, characterized in that the bone support face of each prosthetic web (1, 11, 5, 15) and/or of each support plate (7) is coated with a hydroxyapatite layer.

19. Vertebral articular prosthesis, characterized in that it comprises a first prosthetic element according to one of the claims 1 to 17, which is adapted to be anchored on the upper vertebra of the joint to be treated, wherein said upper prosthetic element comprises a lower prosthetic web (11) having a lower artificial articular surface (12), and a second prosthetic element according to one of the claims 1 to 17, adapted to be anchored on the lower vertebra of the joint to be treated, wherein said prosthetic element comprises an upper prosthetic web (1) having an artificial articular surface (2), said lower and upper artificial articular surfaces having conjugated shapes adapted to form the joint between both vertebras.

## Patentansprüche

1. Prothetisches Element für einen Wirbelgelenkfortsatz, dadurch gekennzeichnet, daß es die folgende Merkmale aufweist:
- mindestens eine hintere Trageplatte (7), so angepaßt, daß sie starr in einer Position, in welcher sie gegen eine Strecke von mindestens eines hinteren Wirbelbogens des Wirbelknochens herausragt, an einem Wirbelknochen verankert ist,
- mindestens eine prothetische Klinge (1, 11, 5) für eine Gelenkfacette, die entweder die gesamte Ersetzung des hinteren Wirbelgelenkfortsatzes (3, 13), an welcher es zur Abstützung angebracht ist, einschließt oder nicht,
und daß jede, von der Trageplatte (7) getragene, prothetische Klinge (1, 11) von der Trageplatte (7) in die Position der natürlichen, durch die Klinge ersetzten Gelenkfacette hinausragt und eine künstliche Gelenkfläche (2, 12) aufweist, deren Form, Position und Ausrichtung der natürlichen, durch die Klinge ersetzten Gelenkfacette (3, 13) entsprechen.

2. Element gemäß Anspruch 1, dadurch gekennzeichnet, daß es mindestens eine, die obere, mit einer flachen oder rückwärts gerichteten, konkaven Gelenkfläche (2) ausgestattete, zur Ersetzung einer oberen Gelenkfacette angepaßte prothetische Klinge (1) tragende Trageplatte (7) und/oder eine untere, mit einer flachen oder vorwärts gerichteten, konvexen Gelenkfläche (12) ausgestattete, zur Ersetzung einer unteren Gelenkfläche angepaßte prothetische Klinge (11) aufweist.

3. Element gemäß einem der Ansprüche 1 und 2, dadurch gekennzeichnet, daß es mindestens eine, zur Abstützung gegen ein Reststück eines Gelenkfortsatzes angepaßte, prothetische Klinge (1, 11) umfaßt, wobei die prothetische Klinge eine Fläche hat, welche gegenüber der Gelenkfläche (2, 12) liegt, die gegen eine Reststrecke des entsprechenden Gelenkfortsatzes (3, 13) herausragt.

4. Element gemäß einem der Ansprüche 1 bis 3, dadurch gekennzeichnet, daß es mindestens eine prothetische Klinge (5, 15) zur völligen Ersetzung eines Wirbelgelenkfortsatzes (3, 13) umfaßt, wobei die Klinge (5, 15) die Form einer Tuberosität mit einer künstlichen Gelenkfläche (2, 12), nämlich ein knolliger Höcker, zur Bildung, wie bei dem natürlichen, durch die prothetische Klinge ersetzten Gelenkfortsatz, eine hintere, externe Begrenzung für den Rückenmarkskanal und eine vordere Begrenzung für das Foramen hat.

5. Element gemäß einem der Ansprüche 1 bis 4, dadurch gekennzeichnet, daß jede prothetische Klinge (1, 11, 5, 15) mittels einer ungestielten Basis (6, 16) mit der Trageplatte (7) verbunden ist, wobei die Einheit, die die prothetische Klinge (1, 11, 5, 15), die Tragefläche (7) und die ungestielte Basis (6, 16) umfaßt, aus einem einzigen, festen, aus biokompatiblen Material gefertigten Teil besteht.

6. Element gemäß einem der Ansprüche 1 bis 5, dadurch gekennzeichnet, daß jede Trageplatte (7) eine konvexe Fläche aufweist, die zum Aufnahme und zum Eingriff und zur Unterstützung durch die konkave Oberfläche des hinteren Wirbelbogens eingerichtet ist und wobei jede Tragefläche (7) eine dünne Platte ist, die so ausgerichtet ist, daß sie die angrenzenden, natürlichen Organe, insbesondere die angrenzenden Muskeln und Bänder nicht behindert.

7. Element gemäß einem der Ansprüche 1 bis 6, dadurch gekennzeichnet, daß an jeder Seite des Dornfortsatzes (9), an der eine Trageplatte (7) angebracht ist, die Mittel zur festen Verankerung der Trageplatte (7) angepaßt sind, um die Trageplatte (7) gegen den hinteren Wirbelbogen des Wirbelknochens zu verankern,
- entweder in Relation zu dem Stiel (10) der gegenüberliegenden hinteren Wirbelbogen,
- oder in Relation zu dem Stiel (10) und auf einem Querfortsatz (8) oder einem Dornfortsatz (9) des Wirbelknochens,
- oder an einem Querfortsatz (8) und an dem Dornfortsatz (9),
- oder an einem Querfortsatz(8), an dem Dornfortsatz (9) und in Relation zu dem Stiel (10) des gegenüberliegenden, hinteren Wirbelbogens.

8. Element gemäß einem der Ansprüche 1 bis 7, dadurch gekennzeichnet, daß es eine einseitige Trageplatte (7) aufweist, die nur an einer Seite des Dornfortsatzes (9) des Wirbelknochens herausragt.

9. Element gemäß einem der Ansprüche 1 bis 7, dadurch gekennzeichnet, daß es eine zweiseitige Trageplatte (7) aufweist, die an beiden Seiten des Dornfortsatzes (9) des Wirbelknochens herausragt.

10. Element gemäß einem der Ansprüche 1 bis 8, dadurch gekennzeichnet, daß es zwei Trageplatten (7), jede an einer Seite des Dornfortsatzes (9) des Wirbelknochens aufweist.

11. Element gemäß Anspruch 10, dadurch gekennzeichnet, daß beide Trageplatten (7) gegenüber dem Dornfortsatz (9) herausragen und darin mittels üblicher Verankerungsmittel (21, 22) verankert sind.

12. Element gemäß einem der Ansprüche 1 bis 11, dadurch gekennzeichnet, daß es Mittel zur festen Verankerung einer Trageplatte (7) auf einem Querfortsatz (8) umfaßt, wobei die Mittel sich mindestens aus einem Haken (28) und/oder mindestens einer Klemme (33) und/oder mindestens einem Kragen (39) zur Klemmung der gegenüber diesem Querfortsatz (8) herausragenden Trageplatte (7) zusammensetzt.

13. Element gemäß Anspruch 12, dadurch gekennzeichnet, daß die Mittel zur Verankerung der Trageplatte (7) auf dem Querfortsatz (8) mindestens einen über eine Achse (30, 36, 37) an der Trageplatte (7) drehbar beweglichen Haken (28, 24, 35) umfaßt, wobei der bewegliche Haken (28, 34, 35) eine Klemmschraube (32, 42, 43) hält, die in ein Gewinde am Haken (28, 34, 35) eingreift, wobei die Schraube angepaßt ist, um direkt oder indirekt auf der Trageplatte (7) oder einem anderen Haken gehalten zu werden, zur Drehung des beweglichen Hakens (28, 34, 35) um die Achse (30, 36, 37) in die Richtung des Klemmens deren freien Endes an die entsprechende Stelle des Wirbelknochens.

14. Element gemäß Anspruch 13, dadurch gekennzeichnet, daß die Verankerungsmittel mindestens eine Klammerklemme (33) umfassen, welche aus zwei Haken (34, 35) gebildet ist und deren gegenüberliegenden; freien Enden (44, 45) in Richtung aufeinander geklemmt sind.

15. Element gemäß einem der Ansprüche 1 bis 11, dadurch gekennzeichnet, daß es Mittel aufweist zur festen Verankerung einer Trageplatte (7) auf einem Dornfortsatz (9) umfaßt, wobei die Mittel mindestens einen Kragen (21) und/oder mindestens eine Flansch (22) zur Klemmung der gegenüber dem Dornfortsatz (9) herausragenden Trageplatte (7) aufweisen.

16. Element gemäß einem der Ansprüche 1 bis 15, dadurch gekennzeichnet, daß jede Trageplatte (7) Mittel (49) zur Kopplung der rückgrätigen Instrumentation hält.

17. Element gemäß einem der Ansprüche 1 bis 16, dadurch gekennzeichnet, daß die Knochentragefläche jeder prothetischen Klinge (1, 11, 5, 15) und/oder jeder Trageplatte (7) aus einem mit verteilten Spitzen ausgestatteten porösen Metall gebildet ist.

18. Element gemäß einem der Ansprüche 1 bis 17, dadurch gekennzeichnet, daß die Knochentragefläche jedes prothetischen Klinge (1, 11, 5, 15) und/oder jeder Trageplatte (7) mit einer Hydroxyapatitschicht überzogen ist.

19. Wirbelgelenkprothese, dadurch gekennzeichnet, daß sie ein erstes prothetisches Element gemäß einem der Ansprüche 1 bis 17 aufweist, das angepaßt ist, um auf dem oberen Wirbelknochen des zu behandelnden Gelenks verankert zu werden, wobei dieses obere prothetische Element eine untere prothetische Klinge (11) mit einer unteren künstlichen Gelenkfläche (12) aufweist, sowie ein zweites prothetisches Element gemäß einem der Ansprüche 1 bis 17, das angepaßt ist, um auf dem unteren Wirbelknochen des zu behandelnden Gelenks verankert zu werden, wobei dieses prothetische Element eine obere prothetische Klinge (1) mit einer künstlichen Gelenkfläche (2) aufweist, wobei die oberen und unteren künstlichen Gelenkflächen zugeordnete Formen aufweisen, die zur Bildung des Gelenks zwischen beiden Wirbelknochen angepaßt sind.
